(19) Europäisches Patentamt
European Patent Office
Office européen des brevets

(11) Veröffentlichungsnummer: **0 261 185 B1**

(12) **EUROPÄISCHE PATENTSCHRIFT**

(45) Veröffentlichungstag der Patentschrift: **05.08.92**

(51) Int. Cl.$^5$: **C07D 307/935**, A61K 31/557, //C07C405/00

(21) Anmeldenummer: **87902056.8**

(22) Anmeldetag: **25.03.87**

(86) Internationale Anmeldenummer:
**PCT/DE87/00132**

(87) Internationale Veröffentlichungsnummer:
**WO 87/05900 (08.10.87 87/22)**

# (54) 7-OXOPROSTACYCLINDERIVATE UND VERFAHREN ZU IHRER HERSTELLUNG.

(30) Priorität: **26.03.86 DE 3610556**

(43) Veröffentlichungstag der Anmeldung:
**30.03.88 Patentblatt 88/13**

(45) Bekanntmachung des Hinweises auf die Patenterteilung:
**05.08.92 Patentblatt 92/32**

(84) Benannte Vertragsstaaten:
**AT BE CH DE FR GB IT LI LU NL SE**

(56) Entgegenhaltungen:
**EP-A- 0 031 426**
**EP-A- 0 119 949**
**WO-A-82/01002**
**WO-A-85/02187**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **VORBRÜGGEN, Helmut**
**Wilkestr. 7**
**W-1000 Berlin 27(DE)**
Erfinder: **STÜRZEBECHER, Claus-Steffen**
**Kuckucksweg 6**
**W-1000 Berlin 33(DE)**
Erfinder: **MAAS,Manfred**

**(deceased9)(DE)**

**EP 0 261 185 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Prostacyclin-Derivate, Verfahren zu ihrer Herstellung sowie ihre Verwendung als Arzneimittel.

In den europäischen Patentschriften 0 031 426, 0 059 756 und 0 163 672 werden 7-Oxoprostacycline beschrieben, die Blutdruck-senkende, bronchodilatierende und Thrombozytenaggregations-hemmende Eigenschaften besitzen.

Es wurde nun gefunden, daß die Einführung einer Acetylengruppe in 13,14-Stellung und gegebenenfalls zusätzlich einer 3-Oxagruppe nicht zur zu einer bei vergleichbaren Carbacyclinderivaten bereits bekannten Stabilisierung des Prostacyclinmoleküls führt, wobei die Wirkungsdauer der neuen Prostacycline deutlich verlängert wird (vgl. EP-A-0 119 949), sondern ausserdem überraschend zu einer Wirkungsdissoziation.

Die erfindungsgemäßen Verbindungen wirken ähnlich blutdrucksenkend wie Iloprost und aus EP-A-0 163 672 bekannten 7-Oxoprostacyclinderivate, jedoch nicht so stark Thrombozytenaggregations-hemmend wie diese.

Die Erfindung betrifft 7-Oxoprostacycline der Formel I

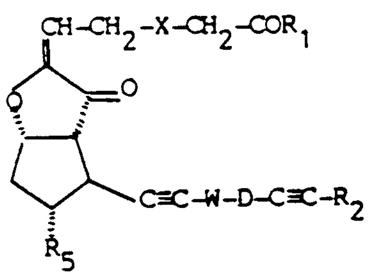

(I),

worin

- $R_1$ den Rest $OR_3$, wobei $R_3$ Wasserstoff oder gegebenenfalls durch Halogen, Phenyl, $C_1$-$C_4$- Alkoxy oder $C_1$-$C_4$-Dialkylamino substituiertes Alkyl mit 1-10 C-Atomen bedeuten kann, oder den Rest $NHR_4$ mit $R^4$ in der Bedeutung eines Alkanoyl- oder Alkansulfonylrestes mit je 1-10 C-Atomen darstellt,
- W eine Hydroxymethylen- oder eine

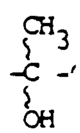

Gruppe, in denen die OH-Gruppe jeweils mit einem Benzoyl- oder Alkansäurerest mit 1-4 C-Atomen verestert oder mit einem Tetrahydropyranyl-, Tetrahydrofuranyl-, ($C_1$-$C_4$-Alkoxy)-$C_1$-$C_4$-alkyl oder Tri-($C_1$-$C_4$-alkyl)-sylylrest veräthert sein kann, wobei die freie OH-Gruppe $\alpha$- oder $\beta$-ständig sein kann,
- D eine geradkettige oder verzweigte Alkylengruppe mit 1-5 C-Atomen,
- $R_2$ eine geradkettige oder verzweigte Alkylgruppe mit 1-6 C-Atomen
- $R_5$ eine Hydroxygruppe, die mit einem Alkansäurerest mit 1-4 C-Atomen verestert oder mit einem Tetrahydropyranyl-, Tetrahydrofuranyl-, ($C_1$-$C_4$-Alkoxy)-$C_1$-$C_4$-alkyl- oder Tri($C_1$-$C_4$-alkyl)-rest veräthert sein kann,
- X ein Sauerstoffatom oder den Rest $-CH_2-$ und, falls $R_3$ Wasserstoff darstellt, deren Salze mit physiologisch verträglichen Basen bedeuten.

Als Alkylgruppe $R_3$ sind gerade oder verzweigte Alkylgruppen mit 1-10 C-Atomen zu betrachten, wie beispielsweise Methyl, Äthyl, Propyl, Butyl, Isobutyl, tert.-Butyl, Pentyl, Neopentyl, Heptyl, Hexyl, Decyl. Die Alkylgruppen $R_3$ können gegebnenfalls 1 bis mehrfach substituiert sein durch Halogenatome, Alkoxygruppen mit 1-4 C-Atomen, und Dialkylamine mit 1-4 C-Atomen. Bevorzugt sind solche Alkylgruppen, die einfach substituiert sind. Als Substituenten seien beispielsweise genannt Fluor-, Chlor- oder Bromatome, Phenyl, Dimethylamin, Methoxy, Äthoxy. Als bevorzugte Alkylgruppen $R_3$ sind solche mit 1-4 C-Atomen,

wie z.B. Methyl, Äthyl, Propyl, Dimethylaminopropyl, Isobutyl und Butyl zu nennen.

Als Säurerest $R_4$ kommen physiologisch verträgliche Säurereste in Frage. Bevorzugte Säuren sind organische Carbonsäuren und Sulfonsäuren mit 1-15 Kohlenstoffatomen, die der aliphatischen, cycloaliphatischen, aromatischen, aromatisch-aliphatischen und heterocyclischen Reihe angehören. Diese Säuren können gesättigt, ungesättigt und/oder mehrbasisch und/oder in üblicher Weise substituiert sein. Als Beispiele für die Substituenten seien Alkyl-, Hydroxy-, Alkoxy-, Oxo- oder Aminogruppen oder Halogenatome erwähnt.

Beispielsweise seien folgende Carbonsäuren genannt: Ameisensäure, Essigsäure, Propionsäure, Buttersäure, Isobuttersäure, Valeriansäure, Isovaleriansäure, Capronsäure, Önanthsäure, Caprylsäure, Pelargonsäure, Caprinsäure, Undecylsäure, Laurinsäure, Tridecylsäure, Myristinsäure, Pentadecylsäure, Trimethylessigsäure, Diäthylessigsäure, tert.-Butylessigsäure, Cyclopropylessigsäure, Cyclopentylessigsäure, Cyclohexylessigsäure, Cyclopropancarbonsäure, Cyclohexancarbonsäure, Phenylessigsäure, Phenoxyessigsäure, Methoxyessigsäure, Äthoxyessigsäure, Mono, Di- und Trichloressigsäure, Aminoessigsäure, Diäthylaminoessigsäure, Piperidinoessigsäure, Morpholinoessigsäure, Milchsäure, Bernsteinsäure, Adipinsäure, Benzoesäure, mit Halogen-, Trifluormethyl-, Hydroxy-, Alkoxy- oder Carboxy-Gruppen substituierte Benzoesäuren, Nikotinsäure, Isonikotinsäure, Furan-2-carbonsäure, Cyclopentylpropionsäure. Als besonders bevorzugte Acylreste werden solche mit bis zu 10 Kohlenstoffatomen betrachtet. Als Sulfonsäuren mit bis zu 10 C-Atomen kommen
beispielsweise Methansulfonsäure,
Äthansulfonsäure, Isopropansulfonsäure, β-Chloräthansulfonsäure, Butansulfonsäure, Cyclopentansulfonsäure, Cyclohexansulfonsäure, Benzolsulfonsäure, p-Toluolsulfonsäure, p-Chlorbenzolsulfonsäure, N,N-Dimethylaminosulfonsäure, N,N-Diäthylaminosulfonsäure, N,N-Bis-(β-chloräthyl)-aminosulfonsäure, N,N-Diisobutylaminosulfonsäure, N,N-Dibutylaminosulfonsäure, Pyrrolidino-, Piperidino-, Piperazino-, N-Methylpiperazino- und Morpholinasulfonsäure in Frage.

Die Hydroxygruppen $R_5$ und in W können funktionell abgewandelt sein, beispielsweise durch Verätherung oder Veresterung, wobei die freien oder abgewandelten Hydroxygruppen in W α- oder β-ständig sein können, und wobei freie Hydroxygruppen bevorzugt sind. Als Äther- und Acylreste kommen die dem Fachmann bekannten Reste in Betracht. Bevorzugt sind leicht abspaltbare Ätherreste wie beispielsweise der Tetrahydropyranyl-, Tetrahydrofuranyl-, α-Äthoxyäthyl-, Trimethylsilyl-, Dimethyl-tert.-butylsilyl- und Tribenzyl-silylrest. Als Acylreste kommen $C_1$-$C_4$-Alkanoylreste wie beispielsweise Acetyl, Propionyl, Butyryl oder Benzoyl in Frage.

Als Alkylgruppen $R_2$ kommen gerad- und verzweigtkettige Alkylreste mit 1-10, insbesondere 1-6 C-Atomen, in Frage. Beispielsweise genannt seien Methyl, Äthyl, Propyl, Butyl, Isobutyl, tert.-Butyl, Pentyl, Hexyl, Heptyl, Octyl.

Als Alkylengruppen D kommen geradkettige oder verzweigtkettige Alkylenreste mit 1-10, insbesondere 1-5 C-Atomen, in Frage. Beispielsweise genannt seien: methylen, äthylen, 1,2-propylen, äthyläthylen, trimethylen, tetramethylen, pentamethylen, 1-methyl-tetramethylen, 1-methyl-trimethylen, 2-methyl-trimethylen, 2-methyl-tetramethylen.

Zur Salzbildung mit den freien Säuren ($R_3$ = H) sind anorganische und organische Basen geeignet, wie sie dem Fachmann zur Bildung physiologisch verträglicher Salze bekannt sind.

Beispielsweise seien genannt: Alkalihydroxide, wie Natrium- und Kaliumhydroxid, Erdalkalihydroxide, wie Calciumhydroxid, Ammoniak, Amine, wie Äthanolamin, Diäthanolamin, Triäthanolamin, N-Methylglucamin, Morpholin, Tris(hydroxymethyl)-methylamin usw..

Die Erfindung betrifft ferner ein Verfahren zur Herstellung der erfindungsgemäßen 7-Oxoprostacycline der Formel I, dadurch gekennzeichnet, daß man in an sich bekannter Weise eine Verbindung der Formel II

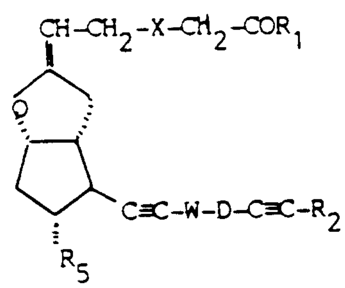

(II),

worin $R_1$, $R_2$, $R_5$, W und D die obenangegebenen Bedeutungen aufweisen, mit Selendioxid oxydiert.

Die Umsetzung der Verbindungen der Formel II
mit Selendioxid wird bei Temperaturen von 20-140° C, vorzugsweise bei 50-120° C, in einem organischen Lösungsmittel, vorzugsweise Dioxan oder tert.-Butanol in 0,5-10 Stunden unter Inertgas (wie z.B. $N_2$ oder Ar) und unter Rühren gegebenenfalls unter Zusatz einer Amin-Base, wie Pyridin oder Hexamethyldisilazan vorgenommen.

Die Verseifung der 7-Oxoprostacyclinester wird nach den dem Fachmann bekannten Methoden durchgeführt, beispielsweise mit basischen Katalysatoren. Die Einführung der Estergruppe, bei welcher $R_3$ eine Alkylgruppe mit 1-10 C-Atomen darstellt, erfolgt nach den dem Fachmann bekannten Methoden. Die Carboxyverbindungen werden beispielsweise mit Diazokohlenwasserstoffen in an sich bekannter Weise umgesetzt. Die Veresterung mit Diazokohlenwasserstoffen erfolgt zum Beispiel dadurch, daß man eine Lösung des Diazokohlenwasserstoffes in einem inerten Lösungsmittel, vorzugsweise in Diäthyläther mit der Carboxyverbindung in dem gleichen oder in einem anderen inerten Lösungsmittel, wie zum Beispiel Methylenchlorid, vermischt. Nach beendeter Umsetzung in 1 bis 30 Minuten wird das Lösungsmittel entfernt und der Ester in üblicher Weise gereinigt. Diazoalkane sind entweder bekannt oder können nach bekannten Methoden hergestellt werden [Org. Reactions Bd. 8, Seiten 389-394 (1954)].

Die 7-Oxoprostacyclin-Derivate der allgemeinen Formel I mit $R_3$ in der Bedeutung eines Wasserstoffatoms können mit geeigneten Mengen der entsprechenden anorganischen Basen unter Neutralisierung in Salze überführt werden. Beispielsweise erhält man beim Lösen der entsprechenden PG-Säuren in Wasser, welches die stöchiometrische Menge der Base enthält, nach Abdampfen des Wassers oder nach Zugabe eines mit Wasser mischbaren Lösungsmittels, zum Beispiel Alkohol oder Aceton, das feste anorganische Salz.

Zur Herstellung eines Aminsalzes, die in üblicher Weise erfolgt, wird die PG-Säure zum Beispiel in einem geeigneten Lösungsmittel, beispielsweise Äthanol, Aceton, Acetonitril, Diäthyläther oder Benzol gelöst und mindestens die stöchiometrische Menge des Amins dieser Lösung zugesetzt. Dabei fällt das Salz gewöhnlich in fester Form an oder wird nach Verdampfen des Lösungsmittels in üblicher Weise isoliert.

Die funktionelle Abwandlung der freien OH-Gruppen erfolgt nach den dem Fachmann bekannten Methoden. Zur Einführung der Ätherschutzgruppen wird beispielsweise mit Dihydropyran in Methylenchlorid, Benzol oder Chloroform unter Verwendung eines sauren Katalysators, wie zum Beispiel $POCl_3$, p-Toluolsulfonsäure oder wasserfreier Mineralsäuren umgesetzt. Das Dihydropyran wird im Überschuß angewandt, vorzugsweise in der 2- bis 10-fachen Menge des theoretischen Bedarfs. Die Umsetzung ist normalerweise bei 0° C bis 30° C nach 15-30 Minuten beendet.

Die Einführung der Acylschutzgruppen erfolgt, indem man eine Verbindung der allgemeinen Formel I in an sich bekannter Weise mit einem Carbonsäurederivat, wie zum Beispiel Säurechlorid, Säureanhydrid u.a., in Gegenwart einer tertiären Aminbase, wie z.B. Pyridin, Dimethylaminopyridin etc. umsetzt.

Die Freisetzung einer funktionell abgewandelten OH-Gruppe zu den Verbindungen der Formel I erfolgt nach bekannten Methoden. Beispielsweise wird die Abspaltung der Ätherschutzgruppen in einer wässrigen Lösung einer organischen Säure, wie zum Beispiel Essigsäure, Propionsäure u.a. oder in einer wässrigen Lösung einer anorganischen Säure, wie zum Beispiel Salzsäure, durchgeführt. Zur Verbesserung der Löslichkeit wird zweckmäßigerweise ein mit Wasser mischbares inertes organisches Lösungsmittel zugesetzt. Geeignete organische Lösungsmittel sind zum Beispiel Alkohole, wie Methanol und Äthanol und Äther, wie Dimethoxyäthan, Dioxan und Tetrahydrofuran. Tetrahydrofuran wird bevorzugt angewendet. Die Abspaltung wird vorzugsweise bei Temperaturen zwischen 20° C und 80° C durchgeführt.

Die Abspaltung der Silylätherschutzgruppen erfolgt beispielsweise mit Tetrabutylammoniumfluorid oder mit KF in Gegenwart eines Kronenäthers. Als Lösungsmittel sind beispielsweise geeignet Tetrahydrofuran, Diäthyläther, Dioxan, Methylenchlorid usw. Die Abspaltung wird vorzugsweise bei Temperaturen zwischen 0° C und 80° C durchgeführt.

Die Verseifung der Acylgruppen erfolgt beispielsweise mit Alkali- oder Erdalkali-carbonaten oder -hydroxyden in einem Alkohol oder der wässrigen Lösung eines Alkohols. Als Alkohole kommen aliphatische Alkohole in Betracht, wie zum Beispiel Methanol, Äthanol, Butanol usw., vorzugsweise Methanol. Als Alkalicarbonate und -hydroxyde seien Kalium- und Natriumsalze genannt, bevorzugt sind jedoch die Kaliumsalze. Als Erdalkalicarbonate und -hydroxyde sind beispielsweise geeignete Calciumcarbonat, Calciumhydroxyd und Bariumcarbonat. Die Umsetzung erfolgt bei -10° C bis 70° C, vorzugsweise bei 25° C.

Die Umsetzung der Verbindung der Formel I mit $R_3$ in der Bedeutung eines Wasserstoffatoms mit einem Isocyanat der allgemeinen Formel

$R_4\text{-}N=C=O$ (V),

worin $R_4$ die obenangegebene Bedeutung hat, erfolgt gegebenenfalls unter Zusatz eines tertiären Amins, wie zum Beispiel Triäthylamin oder Pyridin. Die Umsetzung kann ohne Lösungsmittel oder in einem inerten Lösungsmittel, vorzugsweise Acetonitril, Tetrahydrofuran, Aceton, Dimethylacetamid, Methylenchlorid, Diäthyläther, Benzol, Toluol, Dimethylsulfoxid, bei Temperaturen ober- oder unterhalb Raumtemperatur, zum Beispiel zwischen -80° C bis 100° C, vorzugsweise bei 0° C bis 30° C, vorgenommen werden.

Die als Ausgangsmaterial dienenden Verbindungen der Formel II können beispielsweise hergestellt werden, indem man in an sich bekannter Weise ein bekanntes Prostaglandin F-Derivat der Formel III

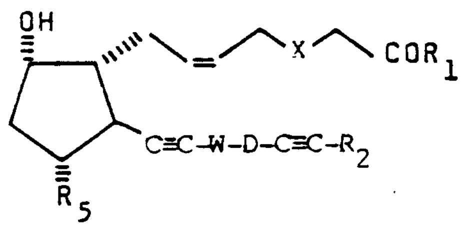

(III),

mit Jod in Gegenwart eines Alkalihydrogencarbonats oder Alkalicarbonats zu den Verbindungen der allgmeinen Formel IV

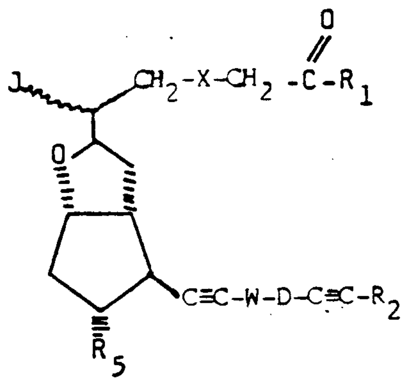

(IV),

umsetzt.[J. Tömösközi et al., Tetrahedron Letters, 2627 (1977)].

Anschließend kann man gegebenenfalls freie Hydroxygruppen durch Veresterung, Verätherung oder Silylierung schützen. Je nach der gewünschten Bedeutung der Reste in den Endprodukten der Formel I kann man gegebenenfalls eine Carboxygruppe verestern oder eine Carboxygruppe mit Verbindungen der Formel V umsetzen.

Die Umsetzung der Verbindungen der Formel IV zu den Verbindungen der Formel II kann beispielsweise mit 1,5-Diazabicyclo[3,4,0]nonen-5(DBN) oder 1,5-Diazabycyclo[5,4,0]-undecen-5(DBU) in einem inerten Lösungsmittel wie Benzol, Toluol, Tetrahydrofuran usw. oder mit Natriummethylat in Methanol erfolgen. Die Halogenwasserstoffabspaltung wird bei Temperaturen zwischen 0° C und 120° C, vorzugsweise bei 20-60° C durchgeführt.

Die Verbindungen dieser Erfindung eignen sich zur Therapie von Erkrankungen des cardiovaskulären Systems, des Magens, des Pankreas, der Leber und der Niere. Sie wirken insbesondere blutdrucksenkend und bronchodilatorisch.

Die Dosis der Verbindungen ist 1-1 500 µg/kg/Tag, wenn sie am menschlichen Patienten verabreicht werden. Die Einheitsdosis für den pharmazeutisch akzeptablen Träger beträgt 0,01-100 mg.

Bei intravenöser Injektion an wachen, hypertonen Ratten in Dosen von 5, 20 und 100 µg/kg Körpergewicht zeigen die erfindungsgemäßen Verbindungen eine stärkere blutdrucksenkende und länger anhaltende Wirkung als $PGE_2$ und $PGA_2$, ohne wie $PGE_2$ Durchfälle oder $PGA_2$ kardiale Arrhythmien auszulösen.

Bei intravenöser Injektion an narkotisierten Kaninchen zeigen die erfindungsgemäßen Verbindungen in Vergleich zu $PGE_2$ und $PGA_2$ eine stärkere und erheblich länger anhaltende Blutdrucksenkung, ohne daß andere glattmuskuläre Organe oder Organfunktionen beeinflußt werden.

Für die parenterale Verabreichung werden sterile, injizierbare, wässrige oder ölige Lösungen benutzt. Für die orale Applikation sind beispielsweise Tabletten, Dragees oder Kapseln geeignet.

Die Erfindung betrifft damit auch Arzneimittel auf Basis der Verbindungen der allgemeinen Formel I und üblicher Hilfs- und Trägerstoffe.

Die erfindungsgemäßen Wirkstoffe sollen in Verbindung mit den in der Galenik bekannten und üblichen Hilfsstoffen, z.B. zur Herstellung von Blutdrucksenkern dienen.

Beispiel 1

1a) (1S,5R,6R,7R)-6-[(E)-(4S)-2-Brom-3-oxo-4-methyl-non-1-en-6-inyl)-7-benzoyloxy-2-oxabicyclo[3.3.0]-octan-3-on

Eine Lösung von 3,05 g Dimethyl-(2-oxo-3β-methyl-oct-5-inyl)-phosphonat in 30ml wasserfreiem Dimethoxyethan tropft man bei 0°C zu einer Suspension von 575 mg Natriumhydrid/Paraffinölsuspension (50/50) in 50ml Dimethoxyethan. Nach Beendigung der Gasentwicklung wird 30 Min. unter Kühlung gerührt. Dann fügt man 2,205g fein gepulvertes N-Bromsuccinimid zu und setzt das Rühren bei 0- +5° eine weitere Stunde fort. Anschließend versetzt man mit einer Lösung von 2,74g Corey-Lacton in 35ml Dimethoxyethan und rührt weitere 2 Stunden unter Kühlung. Dann gießt man in 1 Liter eiskalte 10%ige Ammoniumchloridlösung und extrahiert 3x mit Ether. Der Extrakt wird 2x mit Wasser gewaschen, mit Magnesiumsulfat getrocknet, und nach Filtrieren im Vakuum vom Lösemittel befreit. Das Rohprodukt wird durch Chromatographie an Kieselgel mit einem Hexan-Ethylacetat Gradientensystem gereinigt. Man erhält 3,01 g der obigen Verbindung = 64 % der Theorie.
IR: 1760 $cm^{-1}$ (3-on), 1720 $cm^{-1}$ (7-benzoat), 1700 $cm^{-1}$ (3-oxo)

1b) (1S,5S,6R,7R)-6-([E]-(3RS,4S)-2-Brom-3-hydroxy-4-methyl-non-1-en-6-inyl-7-benzoyloxy-2-oxabicyclo-[3.3.0]octan-3-on

Zu einer Lösung von 3,01 g des nach obigem Beispiel hergestellten Ketons in 76 ml wasserfreiem Methanol gibt man bei -40°C portionsweise 1,444g Natriumborhydrid. Nach 80 Minuten werden vorsichtig 1,91 ml Eisessig zugetropft. Man verdünnt mit Wasser und extrahiert 3x mit Äther. Den Extrakt wäscht man 1x mit eiskalter Natriumhydrogencarbonatlösung und 2x mit Wasser. Nach Trocknen über Magnesiumsulfat und Filtration wird im Vakuum eingedampft. Durch Chromatographier an Kieselgel mit einem Pentan/Ether Gradientensystem wird das 3-Hydroxygemisch in die Epimeren getrennt. Man erhält 23-30% der Theorie des gewünschten S-Epimer und 60-63% der R-Verbindung. Durch Rückoxydation der letzteren mit Jones-Reagenz und erneute Reduktion kann die Ausbeute an S-Verbindung auf 61% der Theorie gesteigert werden.
IR: 3480 $cm^{-1}$(3-Hydroxy), 1760 $cm^{-1}$(3-on), 1720 $cm^{-1}$(7-benzoat), 1665 $cm^{-1}$(Δ1,2)

1c) (1S,5R,6R,7R)-6-([E]-(3S,4S)-2-Brom-3-hydroxy-4-methyl-non-1-en-6-inyl)-7-hydroxy-2-oxabicyclo[3.3.0]-octan-3-on

1,10 g des 3S-Isomeren aus obigem Beispiel löst man in 9,2 ml wasserfreiem Methylalkohol, fügt 160 mg wasserfreies Kaliumcarbonat zu und rührt utner Argon und Feuchtigkeitsausschluß 3 Stunden bei Raumtemperatur. Nach Abkühlen auf Eisbadtemperatur wird durch Zugabe von 0,16 ml 37%iger Salzsäure neutralisiert. Anschließend wird der Methylalkohol im Vakuum bei max. 30° abdestilliert. Der Rückstand wird mit Dichlormethan versetzt, mit Magnesiumsulfat getrocknet. Der nach Abfiltrieren des Trockenmittels und Entfernen des Lösemittels verbleibende Rückstand wird durch Chromatographie an Kieselgel mit einem Hexan-Aceton-System gereinigt. Die Ausbeute beträgt 775 mg = 90% der Theorie.
IR: 3350 $cm^{-1}$ (OH), 1760 $cm^{-1}$ (3-on)

1d) (1S,5R,6R,7R)-6-([E]-(3S,4S-2-Brom-3-tetrahydropyran-2-yloxy)-4-methyl-non-1-en-6-inyl)-7-(tetrahydropyran-2-yloxy)-2-oxabicyclo-[3.3.0]octan-3-on

Man löst 754 mg des Diols aus obigem Beispiel in 13,8 ml trockenem Dichlormethan, gibt 0,45 ml Dihydrofuran und 3 mg p-Toluolsulfosäure zu und rührt 90 Minuten bei Raumtemperatur. Durch Zugabe einiger Tropfen Triethylamin wird dann die Säure neutralisiert. Die Lösung wird 3x mit Wasser gewaschen, mit Magnesiumsulfat getrocknet und nach Filtrieren im Vakuum vom Lösemittel befreit. Der Rückstand wird durch Chromatographie an Kieselgel mit einem Hexan/Ethylacetat-System gereinigt. Man erhält 1,01 g entsprechend 92% der Theorie.
IR: 1760 $cm^{-1}$(3-on), 1295, 870 und 815 $cm^{-1}$(Teträhydropyranyläther)

1e) (1S,3RS,5R,6R,7R)-6-([E]-(3S,4S)-2-Brom-3-(tetrahydropyran-2-yloxy)-4-methyl-non-1-en-6-inyl)-7-(tetrahydropyran-2-yloxy)-2-oxabicyclo-[3.3.0]-octan-3-ol

Zu einer Lösung von 984 mg der nach vorstehendem Beispiel erhaltenen Verbindung in 23 ml absolutem Toluol tropft man bei -70°C 3,28 ml einer 20%igen Lösung von Diisobutylaluminiumhydrid in Toluol. Nach 30 Minuten zerstört man überschüssiges Hydrid durch Zutropfen von 0,18 ml Propanol-(2). Das Kühlbad wird entfernt, der Ansatz mit 1,64 ml Wasser versetzt und die Mischung solange gerührt, bis sich von den Aluminiumverbindungen absaugen läßt. Man wäscht mit Dichlormethan nach und engt das Filtrat im Vakuum ein. Als Rückstand verbleibt die gewünschte Verbindung in quantitativer Ausbeute.
IR: 3400 $cm^{-1}$(OH), 1295, 870 und 815 $cm^{-1}$ (Tetrahydropyranyläther)

1f) 14-Brom-16$\beta$,20-dimethyl-18,18,19,19-tetradehydro-$PGF_{2\alpha}$-11.15-bis-(tetrahydropyranyläther)

Zu einer Mischung aus 6,38 ml Hexamethyldisilazan und 22 ml wasserfreiem Tetrahydrofuran tropft man bei 0° unter Argonathmosphäre 25,9 ml einer 1,18 molaren Butyllithiumlösung in Hexan. Nach 30 Minuten wird diese Lösung dann zu einer Suspension von 6,75 g Carboxybutyltriphenylphosphoniumbromid in 66 ml wasserfreiem Tetrahydrofuran getropft. Das Salz geht unter Ylidbildung in Lösung. Wenn alles gelöst ist, tropft man eine Lösung von 1,116 g des nach vorstehendem Beispiel erhaltenen Lactols in 18 ml wasserfreiem Tetrahydrofuran flott zu und erwärmt anschließend den Ansatz 90 Minuten auf 40°-45°C. Dann gießt man in 1 Liter eiskalte 10%ige Ammoniumchloridlösung, säuert durch Zugabe von 10%iger Zitronensäurelösung auf pH 4,5 an und extrahiert 4x mit Ether. Der organische Extrakt wird 4x mit je 5 ml 1 n Natronlauge und 1x mit 5ml Wasser extrahiert. Die vereinigten alkalischen Auszüge werden durch Zugabe von 10%iger Zitronensäurelösung wieder angesäuert und wiederum 4x mit Ether extrahiert. Dieser Extrakt wird mit Magnesiumsulfat getrocknet. Der nach Abfiltrieren vom Trockenmittel und Abdampfen des Lösemittels erhaltene Rückstand wird als Rohprodukt weiter umgesetzt.

1g) 14-Brom-16$\beta$,20-dimethyl-18,18,19,19-tetradehydro-PGF-$_{2\alpha}$-methylester-11,15-bis-(tetrahydropyranyläther)

Das in der vorhergehenden Stufe erhaltene Rohprodukt wird in 22ml Dichlormethan gelöst, auf 0°C abgekühlt und mit überschüssiger etherischer Diazomethanlösung versetzt. Nach 10 Minuten wird das überschüssige Diazomethan durch Zugabe von etwas Essigsäure zersetzt. Der nach Verdampfen des Lösemittels erhaltene Rückstand wird durch Chromatographie an Kieselgel mit einem Hexan/Aceton-System gereinigt. Man erhält 976 mg des gewünschten Produkts, entsprechend 83,6% bezogen auf das in der vorhergehenden Stufe eingesetzte Lactol.
IR: 3450$cm^{-1}$ (OH), 1730 $cm^{-1}$(Methylester)

1h) 5-Jod-14-brom-16$\beta$,20-dimethyl-18,18,19,19-tetradehydro-$PGI_1$-methyl-ester-11,15-bis-(tetrahydropyranyläther)

Zu einer Lösung von 952 mg des nach vorstehendem Beispiel erhaltenen Methylester in 17,8 ml Ether gibt man eine Lösung von 1,87 g Natriumhydrogencarbonat in 31,5 ml Wasser. Unter kräftigem Rühren kühlt man auf 0° ab und tropft eine Lösung von 0,79 g Jod in 27ml Ether innerhalb 30 Minuten zu. Man rührt weitere 4 Stunden unter Kühlung. Dann werden im Scheidetrichter die Phasen getrennt. Die etherische wird 1x mit 5%iger Natriumthiosulfatlösung und 2x mit Wasser gewaschen. Nach Trocknen mit Magnesiumsulfat und Abfiltrieren wird das Lösemittel verdampft. Der Rückstand wird durch Chromatographie an Kieselgel mit einem Hexan/Ethylacetat-System gereinigt. Man erhalt 1061 mg der gewünschten Verbindung, entsprechend 93,1% der Theorie.
IR: 1730$cm^{-1}$ (Methylester), 1295, 870 und 815$cm^{-1}$ (Tetrahydropyranyläther)

1i) 16$\beta$,20-Dimethyl-13,14,18,18,19,19-hexadehydro-$PGI_2$-11,15-bis(tetrahydropyranyläther)

300 mg der aus der vorhergehenden Stufe erhaltenen Verbindung werden in einem Gemisch aus 3,84 ml wasserfreiem Dimethylsulfoxyd und 1,61ml wasserfreiem Tetrahydrofuran gelöst. Unter Argonatmosphäre gibt man 220mg Kalium-tert.-butylat zu und rührt 2 Stunden bei Raumtemperatur. Dann wird in 50 ml Eis/Wasser gegossen, die blanke Lösung mit 10%iger Zitronensäurelösung auf pH 6 angesäuert und rasch 3x mit Ether extrahiert. Der Extrakt wird unter Zusatz einiger Tropfen Triäthylamin über Magnesiumsulfat getrocknet. Nach Abfiltrieren wird das Trockenmittel mit Ether/Methanol 1:1 nachgewaschen. Das Filtrat wird

im Vakuum eingeengt und der verbleibende Rückstand als Rohprodukt weiter verarbeitet.

1j) 7-Oxo-16β,20-dimethyl-13,14,18,18,19,19-hexadehydro-$PGI_2$-11,15-bis-(tetrahydropyranyläther)

159 mg Rohprodukt der vorhergehenden Stufe löst man in 6 ml wasserfreiem Dioxan, fügt 0,1 ml Hexamethyldisilazan und 27 mg frisch sublimiertes Selendioxyd zu und erwärmt unter Argonatmosphäre und Rühren auf 100° Badtemperatur. Nach 15 Minuten fügt man 4 ml absolutes tert.-Butanol zu. Nach insgesamt 2 Stunden läßt man auf Raumtemperatur abkühlen, versetzt mit Eiswasser und extrahiert alternierend je 2x mit Ether bzw. Ethylacetat. Die vereinigten Extrakte werden mit Magnesiumsulfat getrocknet. Der nach dem Abfiltrieren und Verdampfen des Lösemittels verbleibende Rückstand wird durch präparative Schichtchromatographie gereinigt. Man erhält nach 2maliger Reinigung 19,7 mg der 7-Ketoverbindung (27,5% der Theorie)
IR: 1740 $cm^{-1}$ (Säure), 1710 $cm^{-1}$ (Keton), 1650 $cm^{-1}$ (Δ5,6)

Als polarere Komponente lassen sich 31,1 mg 5-Oxo-16β,20-dimethyl-6,7-13,14-bis-(didehydro)-18,18,19,19-tetradehydro-$PGI_1$-11,15-bis-(tetrahydropyranyläther) gewinnen (37,4 % der Theorie).
IR: 1740 $cm^{-1}$ (Säure), 1710 $cm^{-1}$ (Keton), 1615$cm^{-1}$ (Δ6,7)

1k) (16S)-7-Oxo-16β,20-dimethyl-13,14,18,18,19,19-hexadehydro-$PGI_2$

38 mg des Bis-tetrahydropyranyläthers aus vorstehendem Beispiel werden 24 Stunden mit 3,8 ml einer Mischung aus 65 Teilen Essigsäure, 35 Teilen Wasser und 10 Teilen Tetrahydrofuran bei Raumtemperatur gerührt. Nach Abdampfen der flüchtigen Anteile im Vakuum bei Raumtemperatur und 2maligem Abdestillieren mit Toluol wird der Rückstand durch präparative Schichtchromatographie gereinigt. Nach erhält 17,9 mg der gewüschten Verbindung (67% der Theorie).
IR: 3400 $cm^{-1}$ (breit) (OH + Säure), 1735 $cm^{-1}$ (Säure), 1725 $cm^{-1}$(7-Keton), 1650 $cm^{-1}$ (Δ5,6)

Beispiel 2

11,15-Bis-(tetrahydropyran-2-yloxy)-16β,20-dimethyl-7-oxo-13,14,18,18, 19,19-hexadehydro-3-oxa-$PGI_2$-tert.-butylester

106 mg 11,15-Bis-(tetrahrydropyran-2-yloxy)-16β,20-dimethyl-13,14,18,18, 19,19-hexadehydro-3-oxa-$PGI_2$-tert.-butylester aus Beispiel 2h) löst man in 5 ml wasserfreiem Dioxan. Nach Zagabe von 29 mg frisch sublimiertem Selendioxyd und 85 mg Hexamethyl-disilazan erwärmt man unter Argon 75 Min. auf 95-100°C. Dann läßt man erkalten, versetzt mit Eiswasser und extrahiert alternierend je 2x mit Äther und Essigester. Die vereinigten Extrakte werden 2x mit Wasser gewaschen, mit Magnesiumsulfat getrocknet und nach Filtrieren vom Lösemittel im Vakuum befreit. Der so erhaltene Rückstand wird durch 2malige präparative Schichtchromatographie gereinigt. Man erhält 26 mg noch verunreinigte Titelverbindung IR 1730$m^{-1}$ (Ester = 7-Oxo), 1670$cm^{-1}$ (5-en)

Daneben werden 8 mg der isomeren Verbindung 11,15-Bis-(tetrahydropyran-2-yloxy)-16β,20-dimethyl-5-oxo-13,14,18,18,19,19-hexadehydro-3-oxa-$PGI_2$-tert.-butylester isoliert.

Das Ausgangsmaterial für Beispiel 2 wurde wie folgt hergestellt:

2a) 14-Brom-11,15-Bis-(tetrahydropyran-2-yloxy)-16β,20 dimethyl-18,18,19,19-tetradehydro-2,3,4-trisnor-$PGF_{2}\alpha$-äthylester

650 mg (1S,5R,6R,7R)-6-[(E)-(2RS,3S,4R)-2-Brom-4-methyl-3-tetrahydropyran-2-yloxy)-non-1-en-6-inyl]-7-(tetrahydropyran-2-yloxy)-2-oxabicyclo[3.3.0]-octan-3-on löst man in 24 ml wasserfreiem Toluol, fügt 810 mg (Ethoxycarbonylmethylen)-triphenylphosphoran zu und rührt unter Argon 48 h bei Raumtemperatur. Nach Abdampfen der Hauptmenge Toluol bei bermindertem Druck gibt man auf eine Kieselgelsäule. Durch Elution mit einem Gradientensystem von Essigester und Hexan erhält man 704 mg Titelverbindung (96% der Theorie).
IR 3450$cm^{-1}$ (9 OH), 1720$cm^{-1}$ (Äthylester), 1640$cm^{-1}$ (Olefin)

$_2$b) 14-Brom-g-tert.-butyl-dimethylsilyloxy-11,15-bis-(tetrahydropyran-2-yloxy)-16β, 20-dimethyl-18,18,19,19-tetradehydro-2,3,4-trisnor-$PGF_{1}\alpha$-äthylester

703 mg der Verbindung nach 2a) löst man in 11,5 ml wasserfreiem Dimethylformamid, gibt 196 mg

Imidazol und 434 mg tert.-Butyl-dimethylchlorsilan zu. Nachdem unter Argon 2,5 h gerührt worden war, wurden weitere 44 mg Imidazol und 109 mg des Chlorsilans zugegeben und der Ansatz über Nacht bei Raumtemperatur stehen gelassen. Dann wird in eiskalte 10%ige Ammonchloridlösung gefallt und 3x mit Äther extrahiert. Die vereinigten organischen Extrakte werden 2x mit Wasser gewaschen, mit Magnesiumsulfat getrocknet. Im Vakuum wird das Lösemittel entfernt und der Rückstand an Kieselgel mit einem Essigester/Hexan-System chromatographiert. Man erhält 750 mg Titelverbindung = 89,9% der Theorie .
IR 1720cm$^{-1}$ (Äthylester), 1640cm$^{-1}$ (Olefin), 840cm$^{-1}$ + 770cm$^{-1}$ (Silyläther)

2c) 14-Brom-9-tert.-butyl-dimethylsilyloxy-11,15-bis-(teträhydropyran-2-yloxyl)-4-hydroxy-16$\beta$,20-dimethyl-18,18,19,19-tetradehydro-1,2,3-trisnor-$PGF_{2\alpha}$

750 mg Verbindung nach 2b) löst man in 13,3 ml wasserfreiem Toluol, leitet Argon über und kühlt auf 0°C ab. Tropfenweise werden 3,75 ml einer 1,2 molaren Lösung von Diisobutylaluminiumhydrid (in Toluol) zugegeben. Anschließend wird 30 Minuten bei 0°C gerührt. Dann tropft man 0,2 ml 2-Propanol und 1,88 ml Wasser zu, entfernt das Kühlbad ung rührt so lange bei Raumtemperatur, bis sich die Reaktionslösung von der ausgefallenen Aluminiumverbindung absaugen läßt. Man wäscht mit Dichlormethan nach und entfernt die Lösemittel im Vakuum. Der Rückstand ( 742mg = 100 % der Theorie) wir ohne Reinigung weiter verarbeitet.
IR 3420cm$^{-1}$ (4 Hydroxy), 830cm$^{-1}$ + 770 cm$^{-1}$ (Silyläther)

2d) 9-tert.-Butyl-dimethylsilyloxy-11,15-bis-(tetrahydropyran-2-yloxy)-4-hydroxy-16$\beta$, 20-dimethyl-13,14,18,18,19,19-hexadehydro-$PGF_{2\alpha}$

742 mg der Verbindung nach 2c) löst man in einem Gemisch aus 5,4 ml wasserfreiem Dimethylsulfoxyd und 2,17 mg wasserfreiem Tetrahydrofuran. Nach Überleiten von Argon fügt man 243 mg Kalium-tert.-butylat zu und rührt 2 h bei Raumtemperatur. Dann versetzt man mit Eiswasser, säuert mit Zitronensäure auf pH 5 an und extrahiert 3x mit Äther:Hexan = 7:3 und 2x mit Äther. Die vereinigten Extrakte werden 1x mit Wasser gewaschen, mit Magnesiumsulfat getrocknet und nach Filtration im Vakuum vom Lösemittel befreit. Der Rückstand wird durch Chromatographie an Kieselgel mit einem Essigester/Hexan-System gereinigt. Man erhält 367mg (59% der Theorie) der gewünschten Verbindung. Durch weitere Elution erhält man 98 mg der 9-Hydroxyverbindung.
IR 3450cm$^{-1}$ (4-Hydroxy), 2250cm$^{-1}$ (13,14 in), 830 + 770cm$^{-1}$ (Silyläther)

2e) 9-tert.-Butyl-dimethylsilyloxy-11,15-bis-(tetrahydropyran-2-yloxy)-16$\beta$,20-dimethyl-13,14,18,18,19,19-hexadehydro-3-oxa-$PGF_{2\alpha}$-tert.-butylester

366 mg der Verbindung nach 2d) werden mit 631 mg Bromessigsäure-tert.-butylester, 1,52 ml 50%iger Kalilauge und 8,5 mg Tetrabutyl-ammoniumhydrogensulfat versetzt und unter einer Argonathmosphäre 2,5 h kräftig gerührt. Man verdünnt mit 2,8 ml Wasser und säuert unter Kühlung durch Zutropfen einer Lösung von 1,35 g Zitronensäuremonohydrat in 2,25 ml Wasser an. Dann wird mit Äther und Wasser versetzt, die Phasen werden getrennt und die wässrige 2x mit Äther extrahiert. Die vereinigten organischen Extrakte werden mit halbkonzentrierter Kochsalzlösung gewaschen und mit Magnesiumsulfat getrocknet. Der nach Filtration und Abdampfen des Lösemittels verbleibende Rückstand wird an Kieselgel mit einem Essigester/Hexan-System chromatographiert. Man erhält 392 mg der Titelverbindung (90% der Theorie)
IR 2260cm$^{-1}$ (13,14 in), 1760 cm$^{-1}$ (Ester), 830 + 770cm$^{-1}$ (Silyläther)

2f) 11,15-Bis-(tetrahydropyran-2-yloxy)-16$\beta$,20-dimethyl-13,14,18,18,19, 19-hexadehydro-3-oxa-$PGF_{2\alpha}$-tert.-butylester

392 mg der Verbindung nach 2e) löst man in 5,4 ml wasserfreiem Tetrahydrofuran, fügt 5,4 ml einer 1molaren Lösung von Tetrabutyl-ammoniumfluorid in Tetrahydrofuran zu und rührt unter einer Argonatmosphäre 20 h bei Raumtemperatur. Es wird mit eiskalter 10%iger Ammoniumchloridlösung versetzt und 2x mit Äther und 1x mit Essigester extrahiert. Die vereinigten Extrakte werden 2x mit Wasser gewaschen. Man trocknet über Magnesiumsulfat, filtriert ab und dampft im Vakuum ein. Der Rückstand wird an Kieselgel chromatographiert. Elution mit einem Aceton/Hexan-System liefert 260 mg (79% der Theorie) der Titelverbindung.
IR 3500cm$^{-1}$ (9-OH), 2250cm$^{-1}$ (13,14-in), 1760cm$^{-1}$ (Ester)

2g) 11,15-Bis-(tetrahydropyran-2-yloxy)-16β,20-dimethyl-5-jod-13,14, 18,18,19,19-hexadehydro-3-oxa-$PGI_1$-tert.-buylester

260 mg der Verbindung nach 2f) löst man in 5,2 ml Äther und versetzt mit einer Lösung von 541 mg Natriumhrogencarbonat in 9,1 ml Wasser. Unter Argon und Eiskühlung tropft man eine Lösung von 230 mg Jod in 7,8 ml Äther zu. Anschließend wird 4 h unter Beibehaltung der Kühlung gerührt. Die Phasen werden dann getrennt, die organische mit eiskalter 5%iger Natriumthiosulfatlösung und 2x mit Wasser gewaschen. Nach Trocknen mit Magnesiumsulfat. Filtrieren und Entfernen des Lösemittels im Vakuum wird der Rückstand durch Chromatographie an Kieselgel mit Hexan/Essigester gereinigt. Man erhält 244 mg (77%) der Titelverbindung und 60 mg der Ausgangsverbindung (23%).
IR 1760 $cm^{-1}$ (Ester)

2h) 11,15-Bis-(tetrahydropyran-2-yloxy)-16β,20-dimethyl-7-oxo-13,14,18,18,19, 19-hexadehydro-3-oxa--$PGI_2$-tert.-butylester

244 mg der Verbindung nach 2g) löst man in 5,6 ml wasserfreiem Benzol, leitet Argon über, gibt 0,56 ml Diazabicyclo-undecen zu und rührt 3 h bei 50-60 °C. Nach dem Abühlen auf Raumtemperatur verdünnt man mit Essigester und wäscht 3x mit Wasser. Die organische Phase wird mit Magnesiumsulfat getrocknet. Nach Filtrieren wird das Lösemittel im Vakuum entfernt. Der Rückstand wird ohne Reinigung weiter verarbeitet.

Beispiel 3

7-Oxo-16β,20-dimethyl-13,14,18,18,19,19,-hexadehydro-3-oxa-$PGI_2$-tert.-butylester

44 mg der Verbindung nach Beispiel 2 werden mit 4,4 ml einer Mischung aus 6,5 ml Essigsäure, 3.5 ml Wasser und 1 ml Tetrahydrofuran 24 h bei Raumtemperatur gerührt. Man entfernt die Essigsäure dich Ahdestillieren im Vakuum bei Raumtemperatur. Nach 2maligem Abdestillieren mit Toluol wird der Rückstand durch präparative Schichtchromatographie gereinigt-Man erhält 32 mg (75% der Theorie) noch leicht verunreinigte Titelverbindung.
IR 3450 $cm^{-1}$ (OH), 1750$cm^{-1}$ (Ester + 7-Oxo), 1670$cm^{-1}$ (5-en)

Beispiel 4

7-Oxo-16β,20-dimethyl-13,14,18,18,19,19-hexadehydro-3-oxa-$PGI_2$

32 mg der Verbindung nach Beispiel 3 löst man in 1,25 ml Methanol, fügt 0,1 ml 10%ige Lithiumhydroxidlösung zu und rührt unter Argonatmosphäre 7 h bei Raumtemperatur. Dann wird durch Zugabe von 29 mg Zitronensäuremonohydrat neutralisiert und das Methanol im Vakuum bei Raumtemperatur entfernt. Der Rückstand wird mehrmals mit einem Dichlormethan-Methanol-Gemisch (7:3) verrieben. Der nach Filtrieren und Verdampfen erhaltene Rückstand wird durch präparative Schichtchromatographie gereinigt. Man erhält 11,7 mg reine Titelverbindung (42 % der Theorie).
IR 3400 $cm^{-1}$ (breit)(OH), 2250$cm^{-1}$(13-14 in), 1740$cm^{-1}$(7-Oxo),1720$^{-1}$ (Säure)

## Patentansprüche

**1.** 7-Oxoprostacyclinderivate der Formel I

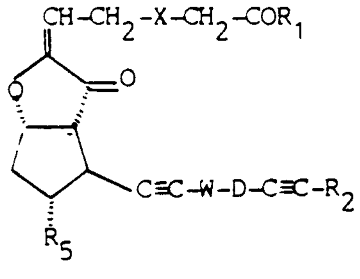

(I),

worin

$R_1$ den Rest $OR_3$, wobei $R_3$ Wasserstoff oder gegebenenfalls durch Halogen, Phenyl, $C_1$-$C_4$-Alkoxy oder $C_1$-$C_4$-Dialkylamino substituiertes Alkyl mit 1-10 C-Atomen bedeuten kann, oder den Rest $NHR_4$ mit $R^4$ in der Bedeutung eines Alkanoyl- oder Alkansulfonylrestes mit je 1-10 C-Atomen darstellt,

W eine Hydroxymethylen- oder eine

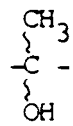

Gruppe, in denen die OH-Gruppe jeweils mit einem Benzoyl- oder Alkansäurerest mit 1-4 C-Atomen verestert oder mit einem Tetrahydropyranyl-, Tetrahydrofuranyl-, ($C_1$-$C_4$-Alkoxy)-$C_1$-$C_4$-alkyl oder Tri-($C_1$-$C_4$-alkyl)-sylylrest veräthert sein kann, wobei die freie OH-Gruppe $\alpha$- oder $\beta$-ständig sein kann,

D eine geradkettige oder verzweigte Alkylengruppe mit 1-5 C-Atomen,

$R_2$ eine geradkettige oder verzweigte Alkylgruppe mit 1-6 C-Atomen

$R_5$ eine Hydroxygruppe, die mit einem Alkansäurerest mit 1-4 C-Atomen verestert oder mit einem Tetrahydropyranyl-, Tetrahydrofuranyl-, ($C_1$-$C_4$-Alkoxy)-$C_1$-$C_4$-alkyl- oder Tri($C_1$-$C_4$-alkyl)-rest veräthert sein kann,

X ein Sauerstoffatom oder den Rest $-CH_2-$ und, falls $R_3$ Wasserstoff darstellt, deren Salze mit physiologisch verträglichen Basen bedeuten.

**2.** (16S)-13,14,18,18,19,19-Hexadehydro-16,20-dimethyl-7-oxo-$PGI_2$.

**3.** (16S)-13,14,18,18,19,19-Hexadehydro-16,20-dimethyl-3-oxa-7-oxo-$PGI_2$.

**4.** Arzneimittel, bestehend aus einer oder mehreren Verbindungen gemäß Anspruch 1 und üblichen Hilfs- und Trägerstoffen.

**5.** Verfahren zur Herstellung von 7-Oxoprostacyclinderivaten der Formel I, dadurch gekennzeichnet, daß man in an sich bekannter Weise eine Verbindung der Formel II,

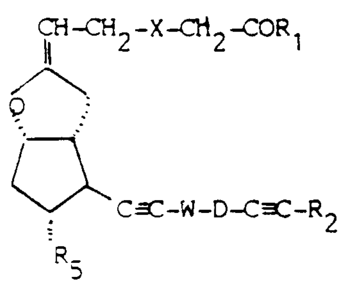

(II),

worin $R_1$, $R_2$, $R_5$, W und D die obenangegebenen Bedeutungen aufweisen, mit Selendioxid oxydiert.

**Claims**

**1.** 7-oxoprostacycline derivatives of formula I

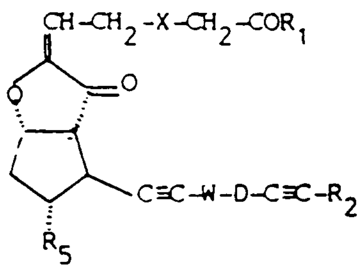

(I)

in which

$R_1$ represents the radical $OR_3$ wherein $R_3$ is hydrogen or alkyl having from 1 to 10 carbon atoms that is optionally substituted by halogen, phenyl, $C_1$-$C_4$-alkoxyor by $C_1$-$C_4$-dialkylamino, or represents the radical $NHR_4$ wherein $R_4$ is an alkanoyl or alkane-sulphonyl radical each having from 1 to 10 carbon atoms,

W represents a hydroxymethylene group or a

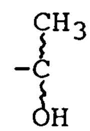

group in each of which the OH group may be esterified by a benzoyl or alkanoic acid radical having from 1 to 4 carbon atoms or etherified by a tetrahydropyranyl, tetrahydrofuranyl, ($C_1$-$C_4$-alkoxy)-$C_1$-$C_4$-alkyl or tri-($C_1$-$C_4$-alkyl)-silyl radical, it being possible for the free OH group to be in the $\alpha$- or $\beta$-configuration,

D represents a straight-chained or branched alkylene group having from 1 to 5 carbon atoms,

$R_2$ represents a straight-chained or branched alkyl group having from 1 to 6 carbon atoms,

$R_5$ represents a hydroxy group which may be esterified by an alkanoic acid radical having from 1 to 4 carbon atoms or etherified by a tetrahydropyranyl, tetrahydrofuranyl, ($C_1$-$C_4$-alkoxy)-$C_1$-$C_4$-alkyl or tri-($C_1$-$C_4$-alkyl) radical,

X represents an oxygen atom or the radical $-CH_2-$, and, when $R_3$ represents hydrogen, the salts thereof with physiologically tolerable bases.

**2.** (16S)-13,14,18,18,19,19-hexadehydro-16,20-dimethyl-7-oxo-$PGI_2$.

**3.** (16S)-13,14,18,18,19,19-hexadehydro-16,20-dimethyl-3-oxa-7-oxo-$PGI_2$.

**4.** Medicament comprising one or more compounds according to claim 1 and customary adjuncts and carriers.

**5.** Process for the preparation of 7-oxoprostacycline derivatives of formula I, characterised in that, in a manner known per se, a compound of formula II

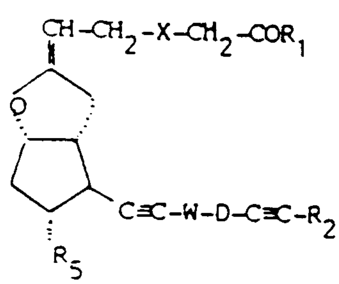

(II),

in which $R_1$, $R_2$, $R_5$, W and D have the meanings given above, is oxidised with selenium dioxide.

## Revendications

**1.** Dérivés de 7-oxoprostacyclines de formule I ci-dessous :

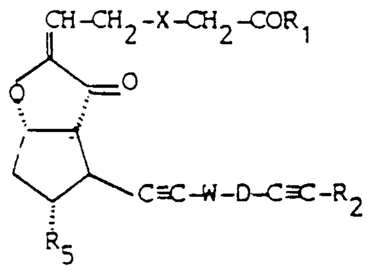

(I),

dans laquelle

- $R_1$ représente un radical $OR_3$, $R_3$ pouvant être l'hydrogène ou un alkyle en $C_1$-$C_{10}$ éventuellement substitué par un halogène, un phényle, un alcoxy en $C_{1-4}$ ou un groupe dialkylamino en $C_{1-4}$, ou bien un radical $NHR_4$, $R_4$ étant un groupe alcanoyle ou alcane-sulfonyle en $C_{1-10}$ chacun,
- W un groupe hydroxyméthylène ou

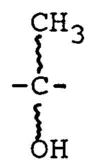

dont le groupe OH peut être estérifié par un radical benzoyle ou alcanoyle en $C_{1-4}$ ou bien éthérifié par un radical tétrahydropyranyle, tétrahydrofuranyle, ($C_1$-$C_4$-alcoxy)-$C_1$-$C_4$-alkyle ou tri-($C_1$-$C_4$-alkyl)-silyle, le groupe OH libre pouvant occuper la position $\alpha$ ou $\beta$,
- D représente un alkylène linéaire ou ramifié en $C_{1-5}$,
- $R_2$ un alkyle linéaire ou ramifié en $C_{1-6}$,
- $R_5$ un hydroxyle pouvant être estérifié par un radical d'alcanoïque en $C_{1-4}$ ou éthérifié par un groupe tétrahydropyranyle, tétrahydrofuranyle, ($C_1$-$C_4$ alcoxy)-$C_1$-$C_4$-alkyle ou tri($C_1$-$C_4$-alkyle), et
- X un atome d'oxygène ou le groupe $-CH_2-$, ainsi que, si $R_3$ est l'hydrogène, les sels de ces composés formés avec des bases physiologiquement tolérées.

**2.** (16S)-13,14,18,18,19,19-hexadéhydro-16,20-dimethyl-7-oxo-$PGI_2$.

**3.** (16S)-13,14,18,18,19,19-hexadéhydro-16,20-diméthyl-3-oxa-7-oxo-$PGI_2$.

**4.** Médicaments formés d'un ou de plusieurs composés de la revendication 1 avec des adjuvants et véhicules courants.

**5.** Procédé de préparation des 7-oxoprostacyclines de formule I selon la revendication 1, procédé caractérisé en ce que l'on oxyde avec du dioxyde de sélénium, d'une manière en elle-même connue, un composé de formule II :

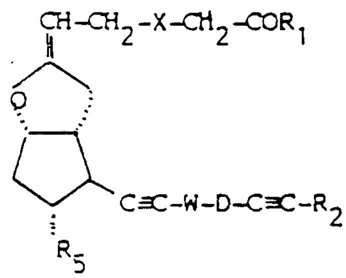

(II),

les divers symboles ayant les significations qui ont été données à la revendication 1.